**Europäisches Patentamt**

**European Patent Office**

**Office européen des brevets**

(19)

(11) **EP 1 083 227 A1**

(12) **EUROPEAN PATENT APPLICATION**

(43) Date of publication:
**14.03.2001 Bulletin 2001/11**

(51) Int Cl.⁷: **C12N 15/63**, C12N 15/86,
A61K 48/00

(21) Application number: **00870183.1**

(22) Date of filing: **24.08.2000**

| | |
|---|---|
| (84) Designated Contracting States:<br>**AT BE CH CY DE DK ES FI FR GB GR IE IT LI LU MC NL PT SE**<br>Designated Extension States:<br>**AL LT LV MK RO SI** | • **Levivier, Marc**<br>**1410 Waterloo (BE)**<br>• **Peschanski, Marc**<br>**94000 Creteil (FR)**<br>• **Brotchi, Jacques**<br>**1180 Brussels (BE)** |
| (30) Priority: **24.08.1999 US 150484 P** | • **Velu, Thierry**<br>**1180 Brussels (BE)** |
| (71) Applicant: **UNIVERSITE LIBRE DE BRUXELLES**<br>**B-1050 Bruxelles (BE)** | (74) Representative: **Van Malderen, Michel et al**<br>**Office van Malderen**<br>**Place Reine Fabiola 6/1**<br>**1083 Bruxelles (BE)** |
| (72) Inventors:<br>• **Tenenbaum, Liliane**<br>**1460 Ittre (BE)** | |

(54) **Antibiotic inducible/repressible genetic construct for gene therapy or gene immunization**

(57)    The present invention is related to an antibiotic inducible / repressible genetic construct (1), controlling the transcription of a gene of interest (2) by a cell (3), said genetic construct comprising a bidirectional controlled activator, responsive promoter /operator sequence (4), located between and controlling the transcription of the gene of interest (2) and of a cistron (6), encoding a reverse antibiotic controlled transactivator (7).

FIG. 6

**Description**

**Field of the invention**

The present invention is in the field of biotechnology and is related to a new antibiotic inducible / repressible genetic construct or system for improving particle the control of gene therapy or gene immunization. In particular, a system or genetic construct adapted for all types of gene therapy and gene immunization based upon the use of naked DNA or DNA incorporated into various vectors (such as plasmids, adeno-associated viruses, autonomous parvoviruses, retroviruses or adenoviruses or a combination thereof).

**Background of the invention**

Various systems comprising naked DNA or DNA incorporated into a suitable vector (plasmid, virus, cationic visicules,...) are used in gene therapy or gene immunization (vaccine). Various publications describe for gene transfer into cells, the use of adeno-associated viruses, which are human defective parvoviruses whose genomes are made of single stranded DNA molecules. Six or five different serotypes have been cloned in prokaryotic plasmids and could be used to derive vectors.

The international patent application PCT/US95/04587 describes gene delivery to adult CNS using AAV vectors.

Humans suffering from Parkinsonism have been treated by striatal implantation of foetal dopaminergic neurons (Lindvall et al., Arch. Neurol. 46:615-631 (1989); Widner et al. New Engl. J. Med. 327: 1556-1563 (1992). Following surgery, the patients exhibited improvement of neurological function. Grafts partially re-establish dopaminergic activity and ameliorate motor functions.
However, the success of foetal brain tissue transplantation into impaired area of Parkinson's and Huntington's patients brain is limited by the poor survival of the graft. To ensure maximal viability, the foetal tissue must be freshly harvested prior to transplantation. Recent advances consist of keeping the tissue refrigerated (at $4°C$) for 24 hours without loss of viability. Nevertheless, the coordination between the harvesting of the foetal tissue and the transplantation procedure is still a problem. Furthermore, the amount of foetal tissue available for transplantation is limited for practical and ethical reasons. Foetal tissue is technically difficult to obtain, particularly if multiple donors are needed for each patient. This limits the widespread applicability of foetal tissue transplantation.

The supply of Glial cell line-Derived Neurotrophic Factor (GDNF), a neurotrophic factor for dopaminergic neurons, could promote the protection of grafted cells as well as of remaining host dopaminergic cells. However, since neurotrophic factors can not cross the brain-blood-barrier, they have to be administrated directly in the brain in sustained levels.

The international patent application PCT/US96/05814 describes a method of using neurotrophic factors to enhance neuronal survival and promote functional integration of grafted neurons using osmotic pumps implanted in the brain. This technique is difficult to implement in the clinics, in particular because of the risk of bacterial contamination.

Improving the survival of the grafted tissue by transfer of genes coding for neurotrophic factors would reduce the amount of tissue needed per patient and make the transplantation therapy available to a greater number of patients.

Stable genetic modification of the graft cells by the means of viral vectors expressing trophic factors could be used to enhance the survival of the grafted tissue.

Genetically-modified foetal mesencephalon fragments or dissociated cell suspensions expressing GDNF could be grafted in order to obtain i) a better survival of the graft (autocrine effect), ii) the protection of host's dopaminergic terminals in the putamen and of dopaminergic cell bodies in the substantia nigra after retrograde transport of GDNF (paracrine effect).

Furthermore, the combination of autocrine and paracrine effects could result in a better correction of parkinsionnian symptoms by foetal grafts transplants.

Adeno-associated virus is a human defective parvovirus whose genome is a single stranded DNA molecule. Five different serotypes have been cloned in prokaryotic plasmids and could be used to derive vectors.

For efficient replication AAV requires a co-infection with a so-called "helper virus", usually adenovirus or herpes simplex virus. In the absence of helper virus, AAV can still enter host cells but it stays latent with his genome integrated in the cellular genome. The genome is flanked by 2 inverted terminal repeats (ITRs) which serve as a replication origin.

The double-stranded form of AAV type 2 has been cloned in a pBR322 plasmid allowing the genetic analysis of the virus as well as the development of vectors for gene transfer (Samulski et al. 1982).

It was soon realized the non-coding ITRs are the only elements required *in cis* for replication and encapsidation of the viral genome (McLaughlin et al., 1987). Accordingly, the vectors derived from AAV only retain the ITRs ; the internal coding region is replaced by the desired transgene(s) and regulatory elements (Samulski et al. 1989). To produce recombinant viral particles, a plasmid containing the ITRs flanking the transgene expression cassette is transfected into producer cells in which AAV rep and cap genes as well as necessary helper virus genes are provided either by transfection or by infection.

AAV vectors transduce various types of neurons in the adult rat (Mc Cown et al., 1997, Klein et al., 1998) and monkey (During et al., 1998) as well rodent and human neurons in culture (Du et al., 1996). Human brain slices from epileptic patients could also be transduced by AAV vectors (Freese et al., 1997). AAV vectors were shown to integrate in neurons (Wu et al., 1998).

The international PCT/US95/04587 describes gene delivery to adult CNS using AAV vectors.

However, controllable gene expression is a prerequisite for safe gene therapy or gene immunization in many protocols: for example, erythropoietin level is critical for the treatment of β-thallassemia.

In models for Parkinson's disease, the intrastriatal delivery of AAV viral vectors encoding GDNF resulting in long-term overexpression of GDNF effectively protects dopaminergic neurons but also results in side-effects on neighboring normal cells (Kirik et al., 2000). PCT/US94/06734 describes a prokaryotic tetracycline system to produce a genetic switch for achieving control of eukaryotic gene expression. In the native prokaryotic tetracycline system, tetracycline is an effector that induces prokaryotic gene expression by binding to a tetracycline repressor protein. In the absence of tetracycline, the tetracycline repressor binds to a tetracycline operator sequence, which is linked to a promoter and represses transcription. In the presence of tetracycline, the tetracylin repressor binds tetracycline, which binding displaces the repressor from the tetracycline operator sequence , so repression is relieved and transcription can begin.

This tetracycline-controlled activator system is constructed by fusing a tetracycline repressor to a transcription activation domain from a protein that activates transcription in eukaryotic cells. In the absence of tetracycline, the tetracycline-controlled activator (tTa) binds the tetracycline operator sequence which is linked to a promoter and activates transcription. In the presence of tetracycline, the tetracycline-controlled activator binds tetracycline, which binding displaces the activator from the tetracycline operator sequence so activation is ended and transcription is silenced. This is a tetracycline-repressible system.

In a further adaptation, the tetracycline-transactivator is mutated in such a way that it binds the tetracycline operator sequence only when tetracycline binds to the mutant tetracycline transactivator (rtTA). Consequently, in the absence of tetracycline, transcription does not occur. In the presence of tetracycline, transcription can begin. This is a tetracycline-inducible system.

These regulatory systems require that two different expression vectors enter each cell. A first expression vector encodes the tetracycline-controlled activator. A second expression vector encodes the desired transgene under the control of the tetracycline operator sequence linked to a promoter.

However, the probability of transfecting a single cell with two plasmid DNAs is significantly lower than for transfecting that cell with one plasmid DNA. Furthermore, it is important for regulation of expression that the two constructs are present in the optimal ratio.

The US 5891718 patent describes a self-accelerating plasmidic system inducible by tetracycline and its analogs, using 2 genes (tTA or rtTA and the gene of interest) expressed from the same tetracycline-inducible / repressible promoter and separated by an "Internal Ribosome Entry Site" (IRES).

This one-plasmid system inducible by tetracylin was shown to be suitable for transient expression in muscle. However, naked DNA is not effective in gene transfer in other organs, for example in the brain. Furthermore, naked DNA does not seem to be effective for long-term gene expression.

In contrast, AAV vectors are effective in long-term gene delivery and expression, in particular in the brain, muscle and liver.

Furthermore a self-accelerating system is based on low level background expression of the tetracycline transactivator and concomitantly of transgene resulting from the basal activity of the minimal CMV promotor (which is part of the tetracycline-responsive element). Therefore, this system is by essence leaky.

Accordingly, the US 5891718 patent describes 40-fold induction of transgene *in vivo* in the muscle. However, background expression in the absence of tetracycline is still detectable in this system.

Bohl et al. (1998) describe a AAV vector expressing erythropoietin (EPO) under a tet-repressible promoter. After injection in the muscle, in the presence of tet EPO is expressed, in the absence of tet EPO is expressed at reduced level but still significantly higher than the background level. In this construct, the reverse tetracycline transactivator (rtTA) is under the control of a constitutive retroviral promoter and EPO is under the control of tetracycline operator (tetO) sequences linked to a minimal CMV promoter (miniCMV). In this system the expression of the EPO transgene is induced ∼10 fold in response to doxycylin.

Haberman et al. (1998) describe a AAV vector expressing gfp under a tet-repressible promoter. This is a a single construct with tTA under control of tetO miniCMV and the reporter gene coding for "green fluorescent protein" (gfp) under the control a second tetO miniCMV. Thus this is a self-accelerating system After infusion in the brain, in the absence of tet, gfp is expressed; in the presence of tet gfp is expressed at reduced level but still significantly higher than the background level. In this system, the addition of tetracycline results in a ∼15-fold reduction of gfp expression.

The presence of a high backgroung level of transgene expression in these two systems could originate from 2 difficulties.

i) Flotte et al. (The Journal of Biol.Chem. 268, 3781-3790, 1993) showed that AAV ITRs have a promoter activity. Thus , in the constructs by Bohl et al. and Haberman et al. tet-independent transcription of gfp can occur from AAV ITRs.
ii) The enhancer elements present in the retroviral LTRs are acting at distance and in both orientations.

Paulus et al. (J.Virol. 70, 62-67, 1996) describe a retroviral vector which proposes a solution for problems n°i) and ii):
They constructed a LTR-TRE-lacZ-IRES-tTA vector in which: "potential cis-regulatory problems in the tetracycline regulation of the phCMV-1 promoter due to proximity to potent Moloney viral enhancer and promoter elements are eliminated by the use of a SIN vector which lacks these elements".
Flotte et al. (ref) showed that AAV ITRs have a promoter activity. Thus , tet-independent transcription of gfp can occur from AAV ITRs.

**Aims of the invention**

The present invention aims to provide a new inducible / repressible genetic construct and a vector comprising it, which do not present the drawbacks of the state of the art and which improve the control of the expression of gene (s) of interest, especially in the field of gene therapy and gene immunization.
A preferred aim of the invention is to provide a single self-accelerating AAV viral vector that contains all the elements necessary for a tight regulation on a single construct smaller than 4.7 kb which can be encapsidated using plasmids expressing capsids from AAV-2 or AAV5 and required adenoviral genes.
A further aim of the invention is to provide strong transcription termination insulating the ITRs from the transgenes and consequently avoiding tet-independent transcription.
It is also an aim of the invention to reduce background expression of the transgene due to basal activity of the bidirectional tetracycline-inducible promoter to level undetectable using quantitative methods known by the one skilled in the art (for example: ELISA, fluorimetry, spectrophotometry, etc.).

**Summary of the invention**

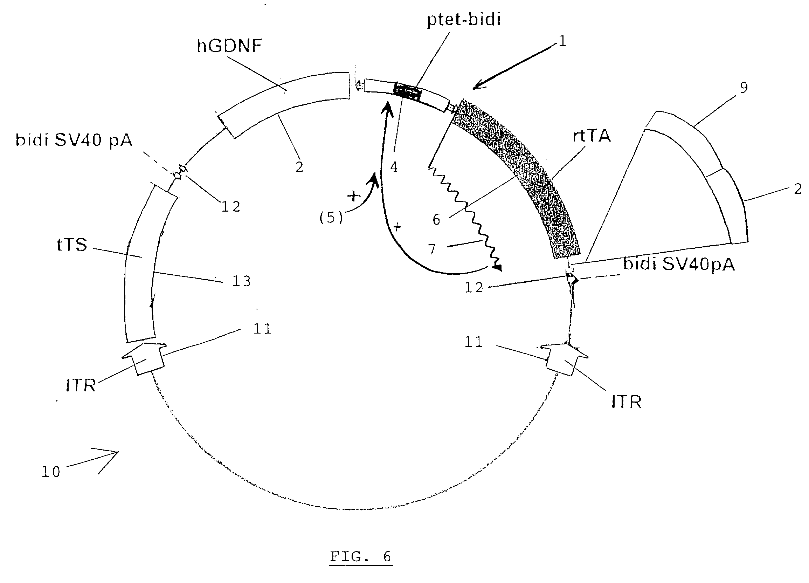

The present invention is related to an antibiotic inducible / repressible genetic construct (or system) for improving the control of gene therapy and gene immunization and which (by the administration of said antibiotic 5 to a patient) induces the transcription and the expression of one (or more) gene(s) of interest incorporated in said genetic construct by a transfected cell or tissue. According to the invention, said genetic construct 1, controlling the transcription of the gene of interest 2 by said cell 3, comprises a bi-directional antibiotic controlled activator-responsive promoter / operator sequence 4 located between (and controlling the transcription of) the gene of interest 2 (or an insertion site for said gene of interest 2) and a cistron 6, encoding a reverse antibiotic controlled transactivator 7. In said construct or system the bi-directional antibiotic controller activator responsive promoter / operator sequence 4 is advantageously activated by the transactivator factor 7, encoded by the reverse antibiotic controlled transactivator nucleotide sequence 6 in the presence of said antibiotic 5 (see figure 6).
Advantageously, the antibiotic elements used according to the invention is the tetracycline or its analogue (such as doxycyclin) and the reverse antibiotic controller activator is a reverse tetracycline controlled transactivator (rtTA), whose sequence has been described by Goossen et al., Science, Vol. 278, p. 1766-1769 (1995) and preferably obtained from the Clontech Laboratories catalogue.
Preferably, the bi-directional antibiotic controller activator responsive promoter / operator sequence 4 comprises, located between two mini CMV-promoter, an antibiotic responsive element, preferably a tetracycline responsive element (TRE), consisting of 7 copies of the 42 base pairs of the tetracycline operator sequence, such as the one described by Baron U. et al., Nucleic acid research, Vol. 17, p. 3605-3606 (1995).
Such bi-directional sequence (Pbi-1) is described in the Clontech Laboratories catalogue.
The genetic construct 1 further comprises (preferably bi-directional) terminator sequences such as (SV40 poly-A) poly-adenylation sequences 12 at its both extremities.
Advantageously, the gene construct 1 according to the invention further comprises downstream the antibiotic controlled transactivator sequence 6, another cistron comprising one (or more) gene(s) of interest 2 (or integration site of one (or more) gene(s) of interest 2) and an internal ribosone entry site (IRES) 9 positioned between said gene of interest 2 or integration site of gene of interest and the second nucleotide sequence 6, encoding the reverse antibiotic controlled transactivator 7.
It is meant by a site of integration of a gene of interest 2, a specific genetic sequence (such as polylinker or a genetic sequence comprising a unique restriction enzyme site) allowing the incorporation of a (foreigner) gene of interest 2 incorporation in the genetic construct 1 according to the invention.

Another aspect of the present invention is related to a vector 10 comprising the genetic construct 1 according to the invention. Preferably, said vector is selected from the group consisting of plasmids, viruses, cationic vesicles or a mixture thereof. Advantageously, the vector 10 comprises the genetic construct 1, disposed between two terminal encapsidation, integration and replication viral genetic sequences 11, preferably between two adeno-associated viral (AAV) or adeno-viral ITR-sequences, two retro-virus LTR-sequences or two palindromic sequences of an autonomous parvovirus.

Preferably, the vector 10 according to the invention comprises also between the genetic construct 1 and said terminal sequence 11, two SV40 bi-directional poly-adenylation (Poly-A) sequences 12.

Furthermore, the nucleic acid construct 1 or the vector 10 according to the invention comprises advantageously a sequence encoding an antibiotic silencer sequence 13 located between the viral terminal sequences 10 and the (bi-directional) SV40 poly-adenylation sequences 12.

In the genetic construct 1 according to the invention, the genes of interest 2 are advantageously therapeutic genes, preferably selected from the group consisting of anti-apoptotic sequences (such as bel-2), sequences encoding neurotrophic factors, preferably GDNF, BDNF, NT4 or CNTF, genetic sequences encoding protein such as erythropoietin (EPO), a human growth factor, a tissue granylocyte macrophage colony stimulator factor (GM-CSF), tissue plasminogen activator (tPA), coagulation factors (FVIII, FIX), insulin, calcitonyn, thimidine kinase, interleukins (IL-2, IL-6,...), interferons ($\alpha$, $\beta$, $\gamma$), tumor necrosis factor, genes encoding enzymes involving the detoxification of free radicals (such as superoxide dismutase or the genetic sequence described in the document PCT/BE98/00124, incorporated herein by reference), genetic sequences encoding tumor-specific antigens such as antigens MAGE 1, MAGE 3, etc.).

Another aspect of the present invention is related to a cell (including stem cells) and/or tissue (preferably a mesencephalic tissue or a striatal tissue) transformed by the genetic construct or the vector according to the invention, preferably a cell which could be used also as a cellular vector or a vaccine for genetic and/or cellular therapy and immunization. Preferably, said genetic therapy using the nucleic acid construct or vector according to the invention allows a transformation of cells obtained from foetal-nervous tissues which are thereafter used for the treatment of neurodigenerative diseases, especially for the treatment of Parkinson disease, Alzheimer or Huntington disease.

Another aspect of the present invention is related also to a method for improving the survival of graft of foetal-nervous tissue by inducing a genetic modification in the cell(s) of said tissue with a viral vector (preferably a AAV-vector, more preferably the vector according to the invention) encoding a neurotrophic factor for improving the treatment of neuro-degenerative diseases, especially Parkinson disease (using for example GDNF) and Huntington disease (using for example CNTF).

More preferably, said neurotrophic factor is the one described above.

## Detailed description of the invention

The inventors have discovered that it is possible to obtain an early and sustained gene transfer into human foetal-nervous tissues by using an AAV-vector, preferably the vector according to the invention.

In this method, tissue fragments rather than dissociated cells are incubated in a viral suspension. AAV vectors expressing the gfp reporter gene transduce human foetal brain tissue with a high efficiency at least for 3 days (the earliest time point tested) to 6 weeks (the latest time point tested).

Unexpectedly, an AAV vector carrying the gene coding for GDNF under the control of an inducible promoter, for example the inducible promoter could be used to transduce human foetal brain tissue prior to transplantation in order to be able to control the amount of GDNF delivered by the grafted tissue.

A similar strategy could be used for the treatment of other neurodegenerative disease, such as e.g. Huntington's disease, using grafts of striatal tissue and an appropriate neurotrophic factor, for example CNTF.

AAV vectors do not express any viral gene and present a minimal risk of immune rejection. Antibodies against AAV capsid have been detected after injection of recombinant virus, however, without concomitant reduction in the number of transduced cells (Lo et al. 1999 ). In the strategy proposed here, foetal tissue is infected ex vivo before transplantation. Washing the tissue to eliminate the excess of virus which did not enter the cells reduces the risk of raising antibodies against capsid proteins by the transplanted host.

In order to obtain a single self accelerating AAV-vector that contains all the elements necessary for tight regulation on a single small construct, the inventors have prepared a genetic construct comprising a bi-directional promoter which drives the transcription of tTA and a gene of interest (GFP or GDNF). This construct is advantageous, because it is small and can be easily encapsidated by using plasmid expressing AAV rep and cap genes and required adenoviral genes.

The construct according to the invention comprises a bi-directional transcription terminal signal (poly-A) located at its both ends (of tTA and of the gene of interest) which provide strong transcription termination insulating the ITRS from transgene and consequently, avoid tet-independent transcription. In addition, the introduction of a transcription silencer sequence (tTS) placed downstream to the ITRs of the virus, provide a constitutive low-level transcription of the tetracycline silencer which reinforces the control of the transgene expression and therefore reduces background expression of said gene of interest due to basal activity of the bi-directional tetracycline inducible promoter / operator sequence to undetectable level (by elisa, fluorimetry, spectrophotometry, etc.).

The tTS sequence consists of a fusion between the procaryotic tetracycline repressor and the silencer domain of the kid-1 eucaryotic transcription factor (Witzgall et al., PNAS 91, p: 4514-4518 (1994)). In this system, in the absence of tetracycline, the tetracylin transactivator does not bind to the tetracycline operator sequence, whereas, the tetracycline silencer binds to the tetracycline operator sequence and blocks transcription. In the presence of tetracycline, the tetracycline transactivator binds to the tetracycline operator sequence and activates transcription whereas the tetracycline silencer detaches from the tetracycline operator sequence.

The present invention would be described in detail in the following examples, which are presented as non-limited illustrations of the various aspects of the present invention.

## Example 1: infection of human foetal mesencephalon with AAV-CMV-EGFP.

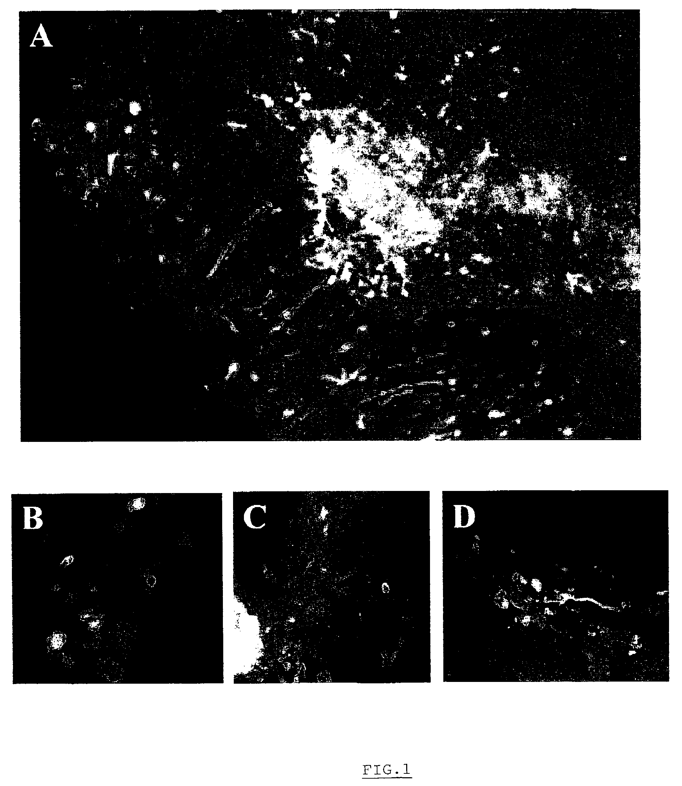

An AAV vector expressing the EGFP gene under the control of the CMV promoter can transfer and express the transgene as early as 3 days and at least until 6 weeks after infection of human foetal mesencephalon. (see fig. 1).

A total of 4 human embryos were infected and gfp expression analyzed.

Embryo 1: 8 weeks of gestation
Embryo 2: weeks
Embryo 3: weeks
Embryo 4: 7 weeks of gestation.

The transduced cells were detected i) by native fluorescence (fig.1 A§D, embryos #2&3) ii) by immunofluorescence (fig.1 B; embryo #1) iii) by immunocytochemistry (fig.1 C; embryo #4).

The mesencephalon was isolated from the rest of the embryo according to (Björklund and Dunnett eds. Neural Transplantation, a practical approach). In a specific embodiment of the invention, small fragments (12 per mesencephalon) are incubated in a viral suspension consisting in either a concentrated purified viral stock-see below) or a purified viral stock diluted in HBSS:organotypic medium (1:1).

After 2 hours to allow viral adsorption and internalization, the fragments were either put in organotypic cultures or dissociated by trypsin and cultured as adherent cells on poly-L-lysin coated slides, a method which allows to select neurons.

In another embodiment, large fragments (half-mesencephalon) are put in organotypic culture and a small volume of a concentrated purified viral stock is injected using a Hamilton syringe.

### *Organotypic cultures of human foetal brain.*

Mesencephalon or adjacent tissue from human embryos (6 to 9 weeks of gestation) was dissected. Large fragments (embryo#1) or small fragments (embryos 2§3) were placed in Transwells (Costar). The lower compartment contained 1 ml of MEM medium supplemented with 25% horse serum, hepes 15 mM, HBSS 25%, glucose 2%, glutamine, $NaHCO_3$ 3.7%, penicillin, streptomycin ("organotypic medium)". The cultures were maintained at 35°C in a humidified atmosphere with 5% $CO_2$.

### *Cultures of human embryonic neurons.*

Infected fragments of mesencephalon were dissociated by trypsin and cultured on squared poly-L-lysin (Sigma)-coated slides (6.25 cm2). (3 slides for one mesencephalon). The cultures were maintained in organotypic medium for 3 days, allowing specific attachment of neurons, for 4 days before fixation with 4% paraformaldehyde.

### *Plasmids*

The AAV vector plasmid pTR-EGFP expressing the EGFP reporter gene (Clonetech) under the control of the CMV promoter, has been described elsewhere (Tenenbaum et al., 1999).

### *Production and purification of recombinant AAV virus.*

Procedure I

Recombinant rAAV-CMV-EGFP virus was obtained as previously described (Tenenbaum et al., 1999). Briefly 293/T cells were co-transfected with pTR-EGFP and pIM45, a plasmid expressing AAV viral genes, cells were infected with adenovirus-2 24 hours post-transfection and harvested 54 hours post-infection. Cesium-chloride purified rAAV-EGFP viral suspension was prepared as previously described.

Procedure II

Recombinant rAAV-EGFPand rAAV-CMV-rGDNF virus were obtained as follows. 293/T cells were co-transfected with pTR-CMV-EGFP or pTR-CMV-rGDNF and pDG, a plasmid expressing AAV viral genes and required adenovirus genes and harvested 52 hours post-transfection. The rAAV viral preparation was purified by iodixanol gradient followed by heparin agarose column according to Zolotukhin et al. (1999, Gene Ther. 6)

### *Fluorescence activated cell sorter (FACS) analysis*

Ten thousand cells were analyzed using a Becton Dickinson FACS scan and the Lysis II software. The statistical analysis of the data was performed using the WINMDI program and the dot-plot option.

### *Titration of rAAV-EGFP virus by FACS analysis.*

Fifty-thousand 293/T cells per well were seeded in 24 wells plates, 1 day before infection. Cells were infected with dilutions of the rAAV-EGFP preparations in 1 ml of serum-free medium containing $10^5$ pfu of wild-type adenovirus. The number of green fluorescent cells expressing the EGFP gene was evaluated 2 days after infection by FACS analysis of $10^4$ cells.

The percentage of fluorescent cells was used to calculate the titer of the rAAV-EGFP viral preparation (expressed in transducing units per ml) as follows:

$$\frac{5\ 104\ X\ \text{percentage of fluorescent cells}}{100X\ \text{dilution of rAAV}}$$

### *Immunofluorescence*

Gfp-immunoflurescence on brain sections:

Foetal samples were washed with PBS at 35°C for 15 min., then 4% paraformaldehyde in PBS at 35°C was carefully added on the sample and the plates were further maintained at 35°C for 15 min. The samples were post-fixed in 4% paraformaldehyde at 4°C overnight, then rinsed with PBS and immersed in PBS 20% sucrose 4% paraformaldehyde overnight. The samples were frozen in a mixture of isopentane and dry ice 30 seconds at -20°C, 10 seconds at -30°C and for overnight at -80°C.
Twenty micron cryostat sections were labeled in liquid ("free-floating" method) as follows. Sections were sequentially incubated in: i) THST (50mM Tris, 0.5 M NaCl, 0.5% triton X100 pH7.6) for 30 min.; ii) polyclonal rabbit anti-gfp (Clonetech) diluted 1:250 in THST overnight at 4°C; iii) donkey anti rabbit-biotin (Amersham) 1:200 in TBS, 1 hour at room temperature; iv) streptavidin coupled with fluorescein, 1:100 in TBS. Sections were mounted on gelatin-coated slides, dehydrated and mounted using Vectashield mounting fluid for fluorescence (NTL laboratories).
Sections were photographed using a Zeiss Axiophot 2 microscope equipped with a U.V. lamp and an FITC filter.

### **RESULTS**

### *Injections of rAAV-EGFP in brain slices.*

Experiment 1:

Roughly circular pieces of human foetal cerebral trunk, adjacent to the ventral mesencephalon, with a surface of approximately 5mm$^2$ and thickness of approximately 200 μm were maintained in Transwells at 35°C, 5% $CO_2$ for 4 hours before infection. Two injections of 1 μl of rAAV-EGFP (prepared according to procedure I) containing 1.2 $10^5$

transducing units were performed. PBS was injected in control samples. The first injection was performed transversal to the plan of the sample, while the second injection was performed perpendicularly to the sample in its middle. A 10µl Hamilton seringe connected with a 26 G needle was used. Injections were performed manually with a speed of 0.2 µl/minute. The needle was withdrawn carefully and slowly. The infected tissue was further cultured for 6 weeks, while replacing half of the medium (0.5 ml) by fresh medium twice a week.

Experiment 2:

Small fragments of human foetal mesencephalon (0.1-1 mm$^3$) were incubated in 96 wells plates in 20 µl of a 1:1 mixture of a viral suspension in PBS (containing 3 x 10$^6$ T.U) and organotypic medium. The rAAV- EGFP stock used was prepared according to procedure I and had titer of 3 x 10$^8$ T.U./ml.
After 2 hours at 37°C, the tissue fragments were transferred to Transwells and maintained in culture at 37°C as described above.

Experiment 3:

Small fragments of human foetal mesencephalon (0.1-1 mm$^3$) were incubated in 96 wells plates in 20 µl of a 1:9:10 mixture of a viral suspension in PBS (containing 1.7 x 10$^6$ T.U) : organotypic medium : HBSS. The rAAV- EGFP stock used was prepared according to rpocedure II and had titer of 1.7 x 10$^9$ T.U./ml.
After 2 hours at 37°C, the tissue fragments were dissociated by trypsin and the cell suspension plated on poly-L-lysin coated slides in serum containing ("organotypic") medium. Three days later, the medium was replaced by serum-free medium and the culture was maintained further for 4 days.

***AAV-mediated gene transfer in human foetal mesencephalon.***

Experiment 1:

Six weeks post-injection the samples were fixed and frozen sections were labeled using anti-gfp antibodies. Figure 1A & D shows that numerous cells with a neuronal morphology appeared. The transduced area covered approximately one third of the sample. A smaller area of transduced cells also appeared in the middle of the sample (Fig. 1D), possibly corresponding to the injection that was performed perpendicularly to the slice.

Experiment 2:

Three days and 2 weeks post-injection, the samples were fixed and frozen sections were observed under fluorescence microscopy to detect native gfp fluorescence. Figure 1B & C shows that labeled cells appeared 3 and 7 days post-infection, respectively. At three days, the cells had the morphology of poorly differentiated neurons with very few and short processes. At 7 days, additional processes appeared, suggesting that rAAV-mediated gene transfer does not interfere with the differentiation of neurons in organotypic culture.
Foetal samples were fixed 3 days (B) , 7 days (C) or 6 weeks (A &D) post-infection and frozen. A & D: Twenty micron cryostat sections were immunolabelled as described in Material and Methods. B & C: Fragments were examined by fluorescence microscopy to detect native fluorescence.

Experiment 3:

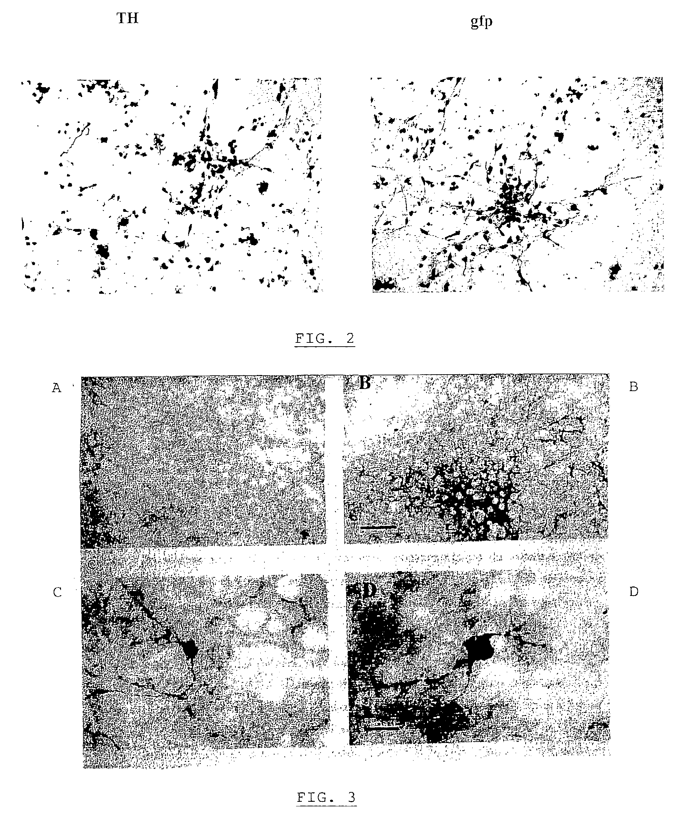

7 days after infection, the cultures were fixed and labeled using anti-gfp polyclonal antibodies or anti-tyrosine hydroxylase polyclonal antibodies (fig.2) or counterstained with hematoxylin to evaluate the total number of cells in the culture.

**Example 2: infection of rat foetal mesencephalon with AAV-CMV-EGFP.**

We found that an AAV vector expressing the EGFP gene under the control of the CMV promoter can transfer and express the transgene as early as 4 days and at least until 3 weeks after infection of rat foetal mesencephalon.
A total of approx.100 rat embryos were used either non-infected , infected with AAV-CMV- Egfp or with AAV-CMV-rGDNF.
The transduced cells were detected i) by native fluorescence ii) by FACS analysis of dissociated cells iii) by immunocytochemistry .
In a specific embodiment of the invention, small fragments (12 per mesencephalon) are incubated in a viral suspension as described in example 1.

***The cultures of rat embryonic neurons and the construction of the vector are described in example 1.***

The AAV vector plasmid expresses the rat GDNF cDNA and expresses the rat GDNF under the control of the CMV promoter. The cDNA coding for rat GDNF was obtained by RT-PCR using mRNA from lesioned sciatic nerve and the following primers:

```
5'ATGAAGCTTGGTCTACGGAGACCGGATCCGAGGTGC_3'
```

and

```
5'GGTCTAGATCTCTGGAGCCAGGGTCAGATACATC 3'.
```

The PCR fragment was digested by HindIII and XbaI cloned in pTR-EGFP.
The resulting clones were sequenced and one clone with a sequence corresponding to rat GDNF cDNA (described in...) was retained.
GDNF expressed by this plasmid after transfection of 293/T cells was measured by ELISA.

*ELISA test*

Recombinant rAAV-EGFP and rAAV-GDNF virus was obtained as follows. 293/T cells were co-transfected with pTR-EGFP and pDG and harvested 52 hours post-infection. The viral suspension were purified by iodixanol gradient followed by heparin agarose column purification as described by Zolotukhin et al. (1999).

***Fluorescence activated cell sorter (FACS) analysis***

Ten thousand cells were analyzed using a Becton Dickinson FACS scan and the Lysis II software. The statistical analysis of the data was performed using the WINMDI program and the dot-plot option.

***Titration of rAAV-EGFP virus by FACS analysis.***

Fifty-thousand 293/T cells per well were seeded in 24 wells plates, 1 day before infection. Cells were infected with dilutions of the rAAV-EGFP preparations in 1 ml of serum-free medium containing $10^5$ pfu of wild-type adenovirus. The number of green fluorescent cells expressing the EGFP gene was evaluated 2 days after infection by FACS analysis of $10^4$ cells.

## RESULTS

Small fragments of rat foetal mesencephalon (12 per mesencephalon) were incubated in Eppendorf tubes in 20 µl of a 1:9:10 mixture of a viral suspension in PBS 1M NaCl (containing $1.7 \times 10^6$ T.U) : organotypic medium : HBSS. The rAAV- EGFP stock used had titer of $1.7 \times 10^9$ T.U./ml.
After 2 hours at 37°C, the tissue fragments were either:

   i) put in organotypic cultures. GFP expression was evaluated either by fluorescence microscopy or by FACS analysis after trypsin dissociation.
   ii) dissociated by trypsin and the cell suspension plated on poly-L-lysin coated slides in serum containing ("organotypic") medium. Three days later, the medium was replaced by serum-free medium and the culture was maintained further for 4 days.

***AAV-mediated gene transfer in rat foetal mesencephalon.***

Experiment 1:

2 days to 3 weeks post-injection the samples were fixed and examined under fluorescence microscopy. This shows that cells with a neuron morphology appeared at 4 days from infection. The transduction efficiency was significantly lower than when using human tissue.

Experiment 2:

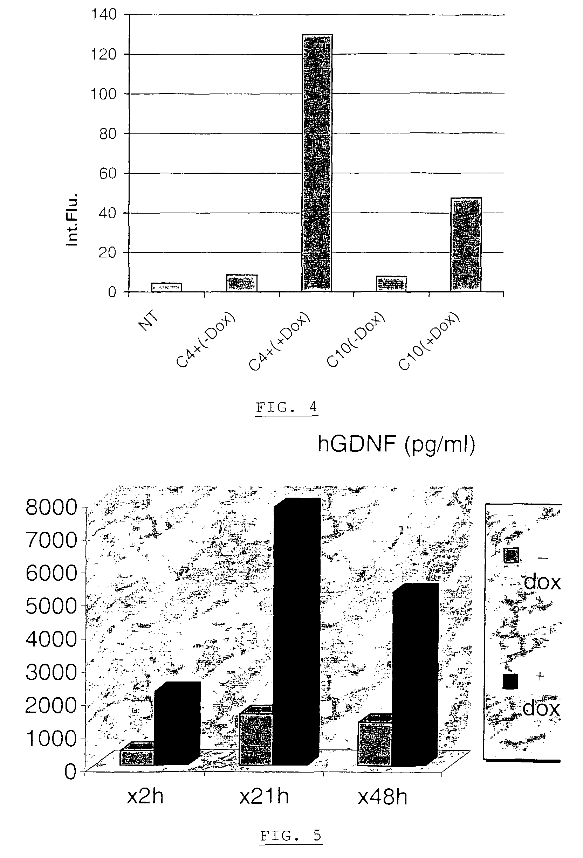

Three days to lmonth post-infection, the samples were dissociated by trypsin and analyzed by FACS . The percentage of gfp-positive cells was low (0.1-0.5%) but stable from 4 days to 1 month post-infection (fig. 3).

Experiment 3:

Rat embryonic mesencephalons were infected either with rAAV-CMV-EGFP or with rAAV-CMV-GDNF and put in organotypic culture. After 3 weeks, the cultures were fixed and immunohistochemistry for dopamine was performed. The density of dopaminergic fibers was higher (A2D) and the dopaminergic cell bodies were larger C2D when the tissue was infected with rAAV-CMV-GDNF than with rAAV-CMV-EGFP.

Experiment 4:

Rat embryonic mesencephalons were infected either with Raav-cmv-egfp or with rAAV-CMV-GDNF. 7 days after infection, the cultures were fixed and incubated with hoechst to detect apoptotic nuclei and labeled using anti-tyrosine hydroxylase polyclonal antibodies followed by a secondary antibody coupled with texas red.

Experiment 5:

Embryo fragments were infected with rAAV-CMV-EGFP, dissociated with trypsin and transplanted in the striatum of adult rats.
Three weeks and 3 months after transplantation, the rats were sacrificed and frozen brain section were submitted to gfp immunohistichemistry.
Three weeks after transplantation, the grafts were clearly visible in the striatum and GFP-positive cells could be detected in the grafts.

**Example 3: Doxycylin-inducible expression of gfp or GDNF mediated by an AAV vector.**

**Material and methods**

*Plasmids*

The minimal CMV promoter is the core immediate early promoter of the human cytomegalovirus, in which the enhancer sites have been deleted.
The bidirectional promoter consists of 2 minimal CMV promoters linked by tetracycline operator sites and is located in the middle of the vector.
Starting from the middle, the right open reading frame directs the transcription of the tTA tetracycline activator consisting in the tetracycline repressor DNA-binding domain fused to the transcription activation domain of the herpes simplex virus VP16 protein.
Also starting from the middle, the left open reading frame directs the transcription of the gfp gene.
Transcription of both cassettes ends at the SV40 bidirectional transcription termination signal.
PTR-tTS-tetON-tTA-EGFP plasmid contains in addition to the 2 cassettes directing the transcription of tTA and EGFP from the bidirectional tetO-miniCMV promoter, the sequence coding for the tetracycline silencer (tTS) under the control of the left ITR. Transcription of the tTS gene ends at the left SV40 bidirectional transcription termination signal which also serves for termination of transcription of the EGFP gene.

*Plasmids*

PTR-tetON-tTA-hGDNF is derived from PTR-tetON-tTA-EGFP. EGFP was replaced by the hGDNF cDNA (gene bank accession n° L19062: nt1-151 and L19063: ntl-485.

*Transfection*

PTR-tetON-tTA-EGFP was transfected into 293T cells using the calcium-phosphate co-precipitation method.

DMEM culture medium containing doxycycline (1 μg/ ml) or not was added to the cells. Fourty-eight hours after transfection, i) the cells were harvested and FACS analyzed or ii) the cells were lyzed and protein extracts were prepared for fluorimetric measure of gfp protein content. Figure 4 shows the data obtained for 2 different pTR-tetONtTA-hGDNF plasmidic clones after transient transfection into 293T cells. The fluorescence intensity was respectively 29.8 fold (C4) and 12.3 fold higher in the presence than in the absence of doxycyclin.

PTR-tetON-tTA-hGDNF was transfected into 293T cells using the calcium-phosphate co-precipitation method. Fourty-eight hours after transfection, the culture medium was replaced by fresh medium and the conditioned medium harvested 2 hrs, 21hrs or 48 hrs after and analysed by ELISA (see fig.5). Figure 7 shows the concentration of GDNF released by the transfected cells in the presence or in the absence of doxycyline. The hGDNF concentration was respectively 5-fold (2hrs), 5-fold (21 hrs) and 4-fold (48 hrs) higher in the presence than in the absence of doxycycline.

**REFERENCES**

Bohl et al., 1998 Blood 92 (5): 1512-1571

Du B, et al. (1996) Gene Ther 3(3):254-61

During MJ, et al. (1998) Gene Ther 5(6):820-7

Fan D, et al. (1998) Neurosci Lett 22;248(1):61-4

Freese A, et al. (1997) Epilepsia 1997 Jul;38(7):759-66

Heberman RP et al., (1998) Gene Ther. 5: 1604-1611

KAPPLITT, M.G. et al. *Nature Genet.* **8** (1994) 148-154.

Kirik et al., 2000 J Neurosci. 20(12):4686-700

Klein et al., 1998 Exp Neurol. 150(2):183-94

Lo, W.D. et al. Hum. Gene Ther. **20** (1999) 201-213.

McCown TJ et al., 1996 Brain Res. 713:99-107

McLaughlin et al., 1988 J. Virol. 62(6):1963-73

Samulski et al., 1982 Proc Natl Acad Sci U S A. 79(6) :2077-81

Samulski et al., 1989 J. Virol. 63:3822-3828

L. Tenenbaum ,M. et al. Gene Ther. **6** (1999) 1045-1053.

Wu P, et al. J Virol 72(7):5919-26

Zolotukhin et al., 1999 Gene Ther. 6(6):973-85

**Claims**

1. Antibiotic inducible / repressible genetic construct (1), controlling the transcription of a gene of interest (2) by a cell (3), said genetic construct comprising a bi-directional controlled activator, responsive promoter / operator sequence (4), located between and controlling the transcription of the gene of interest (2) and of a cistron (6), encoding a reverse antibiotic controlled transactivator (7).

2. The genetic construct according to the claim 1, further comprising a SV40 poly-adenylation sequence (12) at its both extremities.

3. The genetic construct according to any one of the preceding claims characterised in that the antibiotic is the tetracycline or a homologous antibiotic.

4. The genetic construct according to the claim 3, characterised in that the bi-directional antibiotic controlled activator responsive promoter / operator sequence (4) comprises, located between two mini CMV-promoters, an antibiotic responsive element consisting of 7 copies of 42 base pairs of the tetracycline operator sequence.

5. The genetic construct according to any one of the preceding claims, characterised in that it further comprises downstream the first cistron (6) encoding the antibiotic controlled transactivator (7), a second cistron comprising one (or more) gene(s) of interest (2) and an internal ribosome entry site (IRE 9), positioned between said gene of interest (2) and said first cistron (6).

6. Vector (10) comprising the genetic construct (1) according to any one of the preceding claims.

7. The vector according to the claim 6, characterised in that it is selecting from the group consisting of plasmids, viruses, cationic visicules or a mixture thereof.

8. The vector according to the claim 8 characterised in that it comprises the genetic construct (1) disposing between two terminal encapsidation, integration and replication genetic sequence (11) of a virus selecting from the group consisting of adeno-associated viruses, adeno-viruses or autonomous parvoviruses.

9. The vector according to the claim 8, characterised in that it comprises between the genetic construct (1) and the viral terminal sequences (11), two bi-directional SV40 poly-adenylation sequences (poly-A) (12).

10. The vector according to the claim 9, characterised in that it further comprises a sequence encoding an antibiotic silencer sequence (13), located between the viral terminal sequence (11) and the bi-directional SV40 poly-adenylation sequence (12).

11. The vector according to any one of the preceding claims, characterised in that the gene of interest (2) in the genetic construct (1) are therapeutic genes, preferably selected from the group consisting of anti-aptotic sequences, sequences encoding neurotrophic factors (preferably GDNF, BDNF, NT4 or CNTF) genes encoding enzymes involving the detoxification of free radicals, genetic sequences encoding tumor specific antigens, genetic sequences encoding fusogenic peptides or genetic sequences encoding molecules selecting from the group consisting of erythropoetin, human growth factor, tissue plasminogen activator, granulocyremacrophage colony stimulator factor, coagulation factors, insulin, calcitonyn, thymidine kynase, interleukin or tumor necrosis factor.

12. Cell or tissue transformed by the genetic construct (1) or the vector (10) according to any one of the preceding claims.

13. Pharmaceutical composition, preferably a vaccine, comprising an adequate pharmaceutical carrier and a sufficient amount of the nucleic construct, the cell and/or tissue according to any one of the preceding claims.

14. Use of the nucleic construct, the vector, the cell, the tissue or the pharmaceutical composition according to any one of the preceding claims for the manufacture of a medicament for genetic and/or cellular therapy and/or immunization.

15. Use according to the claim 14 for the prevention and/or the treatment of a neuro-degenerative disease, especially for the treatment of Parkinson disease, Alzheimer disease and Huntington disease.

16. A method for improving the survival of graft obtained from foetal-nervous tissues by obtaining a genetic modification of the cell(s) of said graft by a transfection with an AAV-vector, comprising a genetic sequence encoding a neurotrophic factor, preferably selected from the group consisting of GDNF, BDNF, NT4 or CNTF.

17. A cellular vector made of the cell of said graft and comprising the vector according to the invention, having incorporated an AAV-vector comprising a genetic construct encoding a neurotrophic factor.

**18.** The cellular vector according to claim 17, wherein the cells are obtained from a graft of mesencephalic or striatal foetal-nervous tissue.

FIG.1

TH                                          gfp

FIG. 2

FIG. 3

FIG. 4

hGDNF (pg/ml)

FIG. 5

EP 1 083 227 A1

FIG. 6

**European Patent Office**

**PARTIAL EUROPEAN SEARCH REPORT**   **Application Number**

which under Rule 45 of the European Patent Convention EP 00 87 0183
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | A-MOHAMMADI S ET AL: "Efficient transgene regulation from a single tetracycline-controlled positive feedback regulatory system." GENE THERAPY, vol. 5, no. 1, January 1998 (1998-01), pages 76-84, XP000891454 ISSN: 0969-7128 * the whole document * --- | 1-14 | C12N15/63 C12N15/86 A61K48/00 |
| Y,D | WO 97 35992 A (VICAL INC) 2 October 1997 (1997-10-02) * the whole document * --- | 1-14 | |
| Y,D | BARON U ET AL: "CO-REGULATION OF TWO GENE ACTIVITIES BY TETRACYCLINE VIA A BIDIRECTIONAL PROMOTER" NUCLEIC ACIDS RESEARCH,GB,OXFORD UNIVERSITY PRESS, SURREY, vol. 23, no. 17, 11 September 1995 (1995-09-11), pages 3605-3606, XP000775822 ISSN: 0305-1048 * the whole document * --- -/-- | 1-14 | **TECHNICAL FIELDS SEARCHED (Int.Cl.7)** C12N |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

Although claim 15 is directed to a method of treatment of the human/animal body (Article 52(4) EPC), the search has been carried out and based on the alleged effects of the compound/composition.

| Place of search | Date of completion of the search | Examiner |
|---|---|---|
| MUNICH | 17 January 2001 | Morawetz, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EP 1 083 227 A1

| European Patent Office | PARTIAL EUROPEAN SEARCH REPORT | Application Number |
| --- | --- | --- |
| | | EP 00 87 0183 |

| DOCUMENTS CONSIDERED TO BE RELEVANT | | | CLASSIFICATION OF THE APPLICATION (Int.Cl.7) |
| --- | --- | --- | --- |
| Category | Citation of document with indication, where appropriate, of relevant passages | Relevant to claim | |
| Y | GOSSEN M ET AL: "TRANSCRIPTIONAL ACTIVATION BY TETRACYCLINES IN MAMMALIAN CELLS" SCIENCE,US,AMERICAN ASSOCIATION FOR THE ADVANCEMENT OF SCIENCE,, vol. 268, 23 June 1995 (1995-06-23), pages 1766-1769, XP000606285 ISSN: 0036-8075 * the whole document * --- | 1-14 | |
| Y,D | WO 96 33731 A (REGENERON PHARMA) 31 October 1996 (1996-10-31) * the whole document * --- | 15-18 | |
| Y | KLEIN R L ET AL: "Recombinant adeno -associated virus ( AAV ) vectors for brain-derived neurotrophic factor (BDNF) and growth-associated protein ( GAP - 43 ) gene delivery" SOCIETY FOR NEUROSCIENCE ABSTRACTS,US,SOCIETY FOR NEUROSCIENCE, vol. 22, no. 1/03, 16 November 1996 (1996-11-16), page 316 XP002088580 ISSN: 0190-5295 * the whole document * --- | 15-18 | TECHNICAL FIELDS SEARCHED (Int.Cl.7) |
| Y | WO 95 28493 A (UNIV YALE ;UNIV ROCKEFELLER (US)) 26 October 1995 (1995-10-26) * the whole document * --- | 15-18 | |
| | -/-- | | |

EPO FORM 1503 03.82 (P04C10)

19

EP 1 083 227 A1

| European Patent Office | PARTIAL EUROPEAN SEARCH REPORT | Application Number EP 00 87 0183 |

## DOCUMENTS CONSIDERED TO BE RELEVANT

| Category | Citation of document with indication, where appropriate, of relevant passages | Relevant to claim |
|---|---|---|
| Y | ROSENBLAD C ET AL: "Glial cell line-derived neurotrophic factor increases survival, growth and function of intrastriatal fetal nigral dopaminergic grafts." NEUROSCIENCE, vol. 75, no. 4, 1996, pages 979-985, XP000973011 ISSN: 0306-4522 * the whole document * | 15-18 |
| P,X | TENENBAUM L ET AL: "AAV-mediated transduction of human fetal brain tissue." SOCIETY FOR NEUROSCIENCE ABSTRACTS, vol. 25, no. 1-2, 1999, page 212 XP000973044 29th Annual Meeting of the Society for Neuroscience, Part 1;Miami Beach, Florida, USA; October 23-28, 1999 ISSN: 0190-5295 * the whole document * | 15-18 |

CLASSIFICATION OF THE APPLICATION (Int.Cl.7)

TECHNICAL FIELDS SEARCHED (Int.Cl.7)

EPO FORM 1503 03.82 (P04C10)

20

## ANNEX TO THE EUROPEAN SEARCH REPORT
## ON EUROPEAN PATENT APPLICATION NO.

EP 00 87 0183

This annex lists the patent family members relating to the patent documents cited in the above-mentioned European search report.
The members are as contained in the European Patent Office EDP file on
The European Patent Office is in no way liable for these particulars which are merely given for the purpose of information.

17-01-2001

| Patent document cited in search report | | Publication date | Patent family member(s) | | Publication date |
|---|---|---|---|---|---|
| WO 9735992 | A | 02-10-1997 | US | 5891718 A | 06-04-1999 |
| WO 9633731 | A | 31-10-1996 | AU | 5576796 A | 18-11-1996 |
| WO 9528493 | A | 26-10-1995 | CA | 2187626 A | 26-10-1995 |
| | | | EP | 0755454 A | 29-01-1997 |
| | | | JP | 10501686 T | 17-02-1998 |

EPO FORM P0459

For more details about this annex : see Official Journal of the European Patent Office, No. 12/82